# EUROPEAN PATENT APPLICATION

(11) **EP 4 112 004 A1**
(43) Date of publication of application: **04.01.2023**
(21) Application number: 21183082.3
(22) Date of filing: 01.07.2021
(51) Int. Cl.: A61F 2/01

(54) **AN EMBOLIC FILTER**

(71) Applicant: Medtronic Inc., Minneapolis, MN 55432 (US)
(72) Inventor: FRAYSSE, Estelle J.G., Minneapolis MN 55432-5604 (US); KNAAP, Niels van der, Minneapolis MN 55432-5604 (US); KLEIJNEN, Patrick, Minneapolis MN 55432-5604 (US)
(74) Representative: Gray, James

(57) **Abstract**

An embolic filter is provided with a support frame and a porous polymeric membrane connected to the support frame. The embolic filter is configured to be movable between a collapsed state and a deployed state where the filter extends, in use, into contact with an inner wall of a patient's vasculature. The support frame of the embolic filter includes a shape memory material such that the support frame acts as a deployment arrangement to move the embolic filter from the collapsed state into the deployed state.

## Description

Catheters have long been used for the treatment of diseases of the cardiovascular system, such as treatment or removal of stenosis. More recently, catheters have been used for replacement of heart valves, in particular, the aortic valve in a procedure sometimes known as transcatheter aortic valve implantation (TAVI). TAVI is now the principal therapeutic option in patients with severe aortic stenosis deemed inoperable or at high surgical risk. Over 300,000 TAVI procedures were performed worldwide in 2018, and the mortality benefits of TAVI in inoperable and high-risk surgical patients have been recognised. However, the benefit of TAVI is attenuated by the occurrence of major disabling stroke which is associated with increased mortality and in the short term reduced quality of life. During TAVI, extensive manipulation of the calcified native valve and aortic wall takes place using large-sized catheters and rigid delivery systems. Subsequently, balloon valvuloplasty, positioning and implanting of the valve, and possibly post- dilation, takes place. Consequently, dislodgement and embolization of aortic debris and crushed calcified native valves seems inevitable and can provoke stroke by reaching brain vessels.

Embolic protection devices, also known as embolic filters, have been developed to reduce the risk of stroke by providing an efficient protection of the entire brain.

There is a need to provide for improved embolic filter devices having improved filtering capabilities whilst maintaining good anchoring on the vessel.

According to an aspect there is provided an embolic filter comprising: a support frame; and a porous polymeric membrane covering the support frame, wherein the embolic filter is configured to be movable between a collapsed state and a deployed state where the filter extends, in use, into contact with an inner wall of a patient's vasculature, wherein the support frame comprises a shape memory material such that the support frame acts as a deployment arrangement to move the embolic filter from the collapsed state into the deployed state.

The support frame may comprise a shape memory alloy or a shape memory polymer.

The embolic filter may comprise a nitinol support frame.

The support frame may comprise a neck portion configured to conform to an external surface of a catheter body, in use.

The support frame may comprise a plurality of flexible petals extending from said neck portion, and wherein a distal end of each of the flexible petals are configured, in use, to contact with an inner wall of a patient's vasculature when in the deployed state.

The support frame may be formed from a laser cut nitinol frame.

The embolic filter may comprise an internal leaflet extending across an interior of the support frame and configured and arranged to define the neck portion.

The support frame may define an inlet region at an upstream end of the embolic filter that is covered with the porous polymeric membrane, and wherein said inlet region tapers towards a neck portion of the support frame, e.g. so as to define a substantially conical inlet region.

The support frame may define an elongate body comprising first and second end regions thereof each region configured, in use, to contact with an inner wall of a patient's vasculature when in the deployed state.

The elongate body may comprise an external surface, said external surface comprising a narrower region between the first and second end regions.

This narrower central region provides an external 'hourglass' shape that provides a reduced area of contact with a wall of a patient's vasculature, which can help to prevent calcification from being dislodged.

A first end or region of the porous polymeric membrane may be attachable to a body of a delivery catheter, in use. A second end or region of the membrane may be attached to the support frame, wherein the support frame comprises a ring, a coil or an arm configured to extend at least partially around a body of a delivery catheter, in use, over which the porous polymeric membrane extends.

The frame may be formed from an elongate tubular braided nitinol mesh connectable to a body of a delivery catheter, in use, and defining an internal volume.

The porous polymeric membrane may be positioned in the internal volume of the elongate braised nitinol mesh, and wherein the porous polymeric membrane may be arranged to cover the braised nitinol mesh at a first, upstream, end region thereof and is connected to said braided nitinol mesh at a second, downstream, end region thereof.

The porous polymeric membrane may comprise an elongate length that is greater than the elongate length of the braised nitinol mesh.

This oversized polymer membrane provides some loose material along its length, which facilitates crimping of the nitinol mesh.

The polymeric membrane may be connected to the support frame or to a body of a delivery catheter by sewing, adhesive or thermobonding.

The porous polymeric membrane may be a biocompatible fabric comprising a pore size of between 100 and 200 nanometers.

According to a second aspect, there is provided an embolic filter assembly comprising: a delivery catheter; and an embolic filter according to the first aspect integrated with the delivery catheter, wherein the embolic filter is deployable from the delivery catheter prior to the delivery of the replacement valve and retrievable to the delivery catheter after delivery of the replacement valve, wherein the filter is retained in the collapsed state by an overlying sleeve.

Relative movement between the sleeve and the filter in a first direction may enable the support structure to move the filter from the collapsed state to the deployed state by the deployment arrangement.

Relative movement between the sleeve and the filter in a second direction, opposite to the first direction, may move the filter from the deployed state back to the collapsed state.

The nitinol frame may be attached to a body of the catheter body by adhesive, sewing, crimping or thermobonding.

The sleeve may be formed by the catheter body.

The delivery catheter may include an elongate catheter body having a distal tip wherein the filter is provided on the elongate catheter body at a location proximal to the distal tip of the delivery catheter.

Embodiments of the present invention will now be described with reference to the accompanying figures in which:
Figure 1 shows a side view of a combined TAVI delivery catheter and embolic filter located within the aorta of a patient;
Figure 2 shows a perspective view of an embolic filter according to an embodiment of the invention;
Figure 3 shows an end view of the filter of figure 2;
Figure 4 shows a cross-sectional side view of an embolic filter according to another embodiment of the present invention located within the aorta of a patient;
Figure 5 shows a perspective view of the embolic filter of figure 4;
Figure 6 shows a cross-sectional side view of an embolic filter according to another embodiment of the present invention located within the aorta of a patient;
Figure 7 shows a perspective view of the embolic filter of figure 6;
Figure 8 shows a cross-sectional side view of an embolic filter according to another embodiment of the present invention located within the aorta of a patient;
Figure 9 shows a side view of the embolic filter of figure 8;
Figure 10 shows a side view of a combined TAVI delivery catheter and embolic filter according to another embodiment of the present invention located within the aorta of a patient, the embolic filter being in an un-deployed state;
Figure 11 shows a side view of the combined TAVI delivery catheter and embolic filter of figure 10, the embolic filter being in a deployed state;
Figure 12 shows a further side view of the combined TAVI delivery catheter and embolic filter of figure 10, the embolic filter being in a deployed state;
Figure 13 shows a cross-sectional view a combined TAVI delivery catheter and embolic filter according to another embodiment of the present invention, the embolic filter being in an un-deployed state;
Figure 14 shows a cross-sectional view of the combined TAVI delivery catheter and embolic filter of figure 13, the embolic filter being in a deployed state;
Figure 15 shows a side view of a combined TAVI delivery catheter and embolic filter according to another embodiment of the present invention, the embolic filter being in a deployed state; and
Figure 16 shows a side view of a combined TAVI delivery catheter and embolic filter according to another embodiment of the present invention located within the aorta of a patient, the embolic filter being in a deployed state.

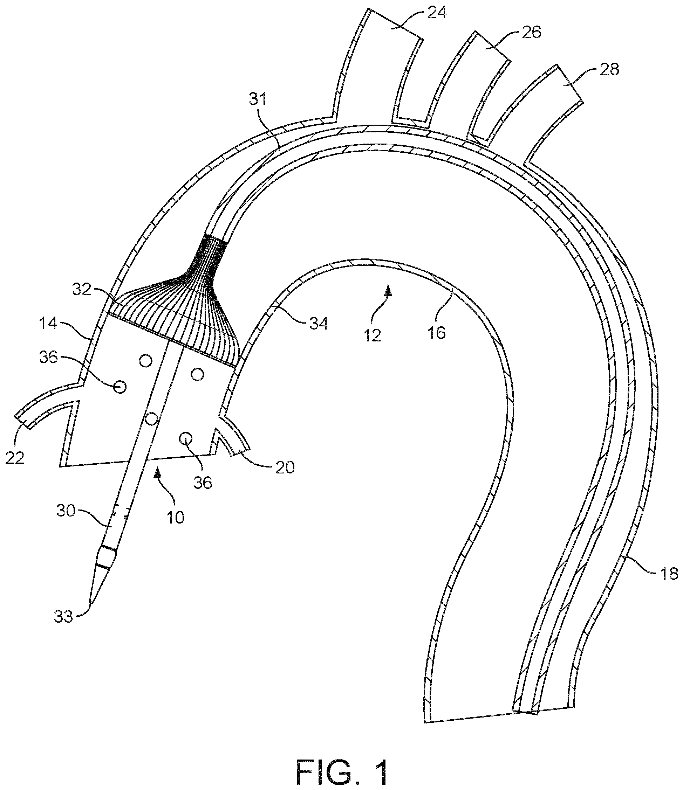

Referring firstly to figure 1 there is shown an embolic filter assembly, generally designated 10. The embolic filter assembly 10 is a combined delivery catheter and embolic filter located within the aorta 12 of a patient. In the illustrated embodiment, the delivery catheter may be considered to be a TAVI delivery catheter, and the embodiment is described with reference to a TAVI procedure. It will be appreciated, however, that in alternative embodiments the delivery catheter may be used for different valve replacement procedures, or may be used in the placement of an intravascular stent. The features of the aorta include the ascending aorta 14, the arch of aorta 16, the descending aorta 18, the left and right coronary arteries 20,22, the brachiocephalic artery 24, the left common carotid artery 26 and the left subclavian artery 28. Not shown is the aortic valve which is to be replaced.

The embolic filter assembly 10 comprises a delivery catheter 30 and an embolic filter 32. The delivery catheter 30 is a TAVI delivery catheter. The delivery catheter 30 may, by way of illustrative example only, correspond to the Medtronic EnVeo PRO ^{™} delivery catheter system. The delivery catheter 30 includes an elongate catheter body 31 having a distal tip 33. The delivery catheter 30 further includes a capsule (not shown) containing a replacement valve. The filter 32 is provided circumferentially around an outer surface of the delivery catheter 30 and extends across the annulus defined the between the delivery catheter 30 and the wall 34 of the ascending aorta 14. As will be described in greater detail below, the filter 32 is configured so as to be able to conform to the wall 34 of the ascending aorta 14 and thus provide required sealing characteristics against the wall of ascending aorta 14. As will also be described in greater detail below, the filter 32 is provided with a high porosity membrane that doesn't restrict blood flow but at the same time provides the desired filtering characteristics to capture any debris 36 that may be dislodged by the TAVI procedure.

In the embodiment shown, the filter 32 is positioned in the ascending aorta 14 downstream of the left and right coronary arteries 20, 22 and upstream of the arch of aorta 16. Any debris 36 that may be dislodged by the TAVI procedure is thus captured by the filter 32 and prevented from reaching and entering the brachiocephalic artery 24, the left common carotid artery 26 and/or the left subclavian artery 28. It will be appreciated that the filter 32 may alternatively be positioned in the arch of the aorta 14 and downstream of the left and right coronary arteries 20,22 and upstream of the brachiocephalic artery 24, the left common carotid artery 26 and/or the left subclavian artery 28. In some arrangements, it will be appreciated that the filter 32 may be arranged downstream of the left and right coronary arteries 20,22 in the arch of the aorta 14 so as to cover the brachiocephalic artery 24, the left common carotid artery 26 and/or the left subclavian artery 28.

Figure 1 shows the filter 32 in a deployed state. The filter 32 is initially provided in an un-deployed or collapsed state whereupon it conforms closely to the outer surface of the delivery catheter 30. The collapsed filter 32 may, for example, be covered by a sheath or sleeve (not shown) that maintains the filter 32 in the collapsed state during advancement of the delivery catheter 30 through the vasculature of the patient. Upon delivery of the replacement valve, i.e. upon positioning of the replacement valve in the desired location within the aorta 12 of a patient, the filter 32 can be moved to the deployed state. This may be achieved by, for example, manipulating the sheath covering the filter 32 in a first direction so as to uncover the filter 32. Put another way, this may be achieved by retracting the sheath relative to the filter 32. The filter 32 may then move from the collapsed state to the deployed state, for example via one or more shape memory portions of the filter 32. The support frame 38 may be formed from a shape memory material, for example a shape memory alloy or shape memory polymer. In each of the embodiments discussed herein, the shape memory material is nitinol, but it will be appreciated that any suitable shape memory material may be used.

With the filter 32 deployed and extending across the aforementioned annulus between the delivery catheter 30 and the wall 34 of the aorta 14, the TAVI procedure can be conducted. As noted above, any debris 36 that is be dislodged by the TAVI procedure is thus captured by the filter 32 and prevented from reaching and entering the brachiocephalic artery 24, the left common carotid artery 26 and/or the left subclavian artery 28.

Once the TAVI procedure has been completed, and prior to the withdrawal of the delivery catheter 30 from the vasculature of the patient, the filter 32 is retrieved while still retaining any debris 36 that may have been collected. Put another way, the filter 32 is moved back to its un-deployed or collapsed state from its deployed state while still retaining any debris 36 that may have been collected. Movement of the filter 32 back to its un-deployed or collapsed state from its deployed state may be achieved by, for example, movement of the sheath is a second direction, opposite to the first direction. Put another way, movement of the filter 32 back to its un-deployed or collapsed state from its deployed state may be achieved by movement of the sheath back to its initial position.

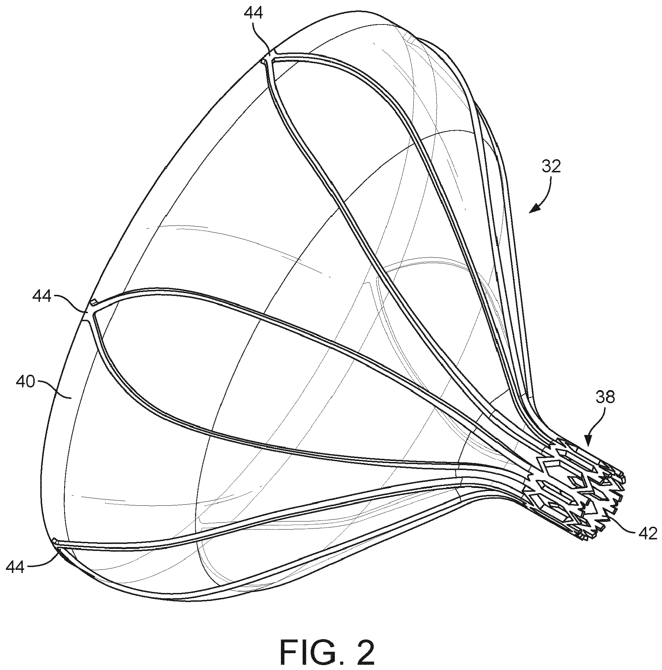

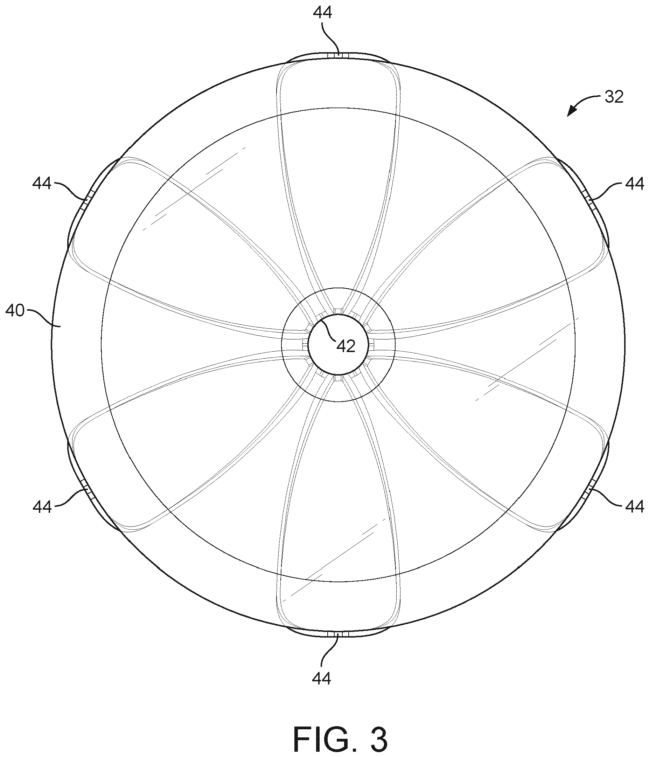

Referring now to figures 2 and 3 there is shown an embodiment of the embolic filter 32. The filter 32 is shown in its deployed state and in isolation, i.e. without the delivery catheter 30. The filter 32 is comprised of a support frame 38. The support frame 38 is formed from a shape memory material, such as a shape memory alloy or a shape member polymer. In the arrangement shown, the support frame 38 is a nitinol support frame. The support frame is a laser cut nitinol alloy frame 38. In an alternative embodiment (not shown) the frame 38 may be formed from a braided nitinol mesh.

The filter 32 also includes a porous polymeric membrane 40 covering the support frame. The polymeric membrane 40 may comprise a biocompatible porous fabric having a pore size of between 100 and 200 nanometers. The frame 38 comprises a neck portion 42 and a plurality of flexible petals 44. In the embodiment shown the filter 32 is provided with six petals 44. It will be appreciated that a greater or lesser number of petals 44 may be used. Each petal 44 is in the form of a loop so as to, in use, minimally occlude the annulus defined between the delivery catheter 30 and the wall 34 of the aorta 12. The polymeric membrane 40 is fixed to the frame 38 by, for example, adhesive.

In use, the collar portion 42 of the frame 38 is axially and rotationally fixed to the shaft of the delivery catheter 30. The petals 44 are biased to the deployed state of the filter 32 shown in Figures 2 and 3 by virtue of the material characteristics of the nitinol alloy. In the collapsed state, the petals 44 are positioned lie against the shaft of the delivery catheter 30 and are retained in this position by an overlying sheath. The sheath can be retracted so as to uncover petals 44 and thus allow petals 44, and filter 32, to move to the deployed state. Movement of the sheath in the direction opposite to that of the retraction direction may be used to move the filter 32 back to the collapsed state, for example once the TAVI procedure has been completed and before the delivery catheter 30 is removed from the patient's vasculature.

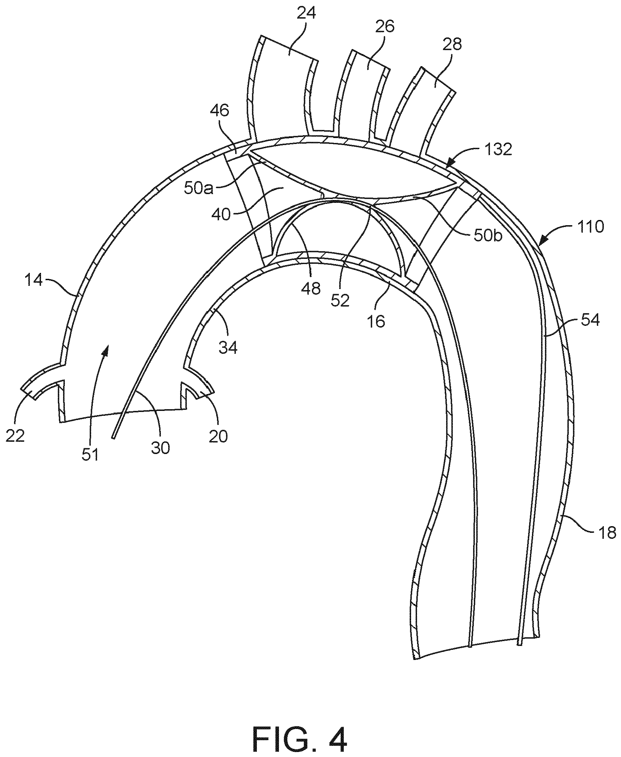

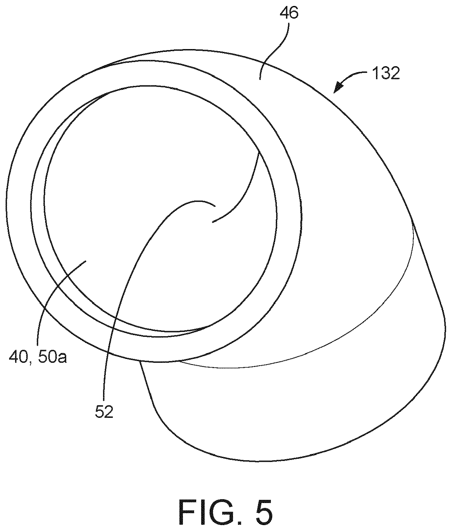

Referring now to figures 4 and 5 there is shown an alternative embodiment of an embolic filter assembly generally designated 110. Features common to the filter assembly 10 described with reference to Figures 1 to 3 are identified with like reference numerals.

The embolic filter assembly 110 includes a delivery catheter 54 and an embolic filter 132. The support frame 38 defines an elongate body 46 defining an external surface thereof. Put another way, the filter 132 comprises an outer tubular portion 46. The filter 132 includes an inner hourglass portion 48 which is located within the body 46. The hourglass portion 48 is also tubular and comprises opposing conical sections 50a, 50b which each narrow towards and are joined by a neck portion 52. The conical sections comprise an upstream conical section 50a and a downstream conical section 50b. In use, the upstream conical section 50a faces the direction of fluid flow (indicated by arrow 51) within the aorta 12. The neck portion 52 is normally closed but can open so as to allow a medical device, such as a TAVI delivery catheter, to pass there through.

In Figure 4, the delivery catheter 54 is separate from the TAVI delivery catheter 30. The TAVI delivery catheter 30 is shown passing through the neck portion 52. The neck portion 52 is configured so as to maintain contact with the TAVI delivery catheter 30 and thus not provide a fluid flow path through the filter 132 between the neck portion 52 and the TAVI delivery catheter 30.

The support frame 38(i.e. the tubular and hourglass portions 46,48) of the filter 132 is formed from braided nitinol mesh. The upstream conical section 50a is furthermore lined with a polymeric membrane 40. The polymeric membrane 40 may comprise a biocompatible porous fabric having a pore size of between 100 and 200 nanometers.

As can be readily seen in figure 4, the outer tubular portion 46 of the filter 132 is curved and is located in, and conforms to the shape of, the arch of aorta 16 in the region of the brachiocephalic artery 24, the left common carotid artery 26 and the left subclavian artery 28. More specifically, the outer tubular portion 46 extends from a location in the arch of aorta 16 that is upstream of the brachiocephalic artery 24 to a location that is downstream of the left subclavian artery 28. It will thus be appreciated that the outer tubular portion 46 overlies the junction of each of the brachiocephalic artery 24, the left common carotid artery 26 and the left subclavian artery 28 with the arch of aorta 16. It will further be appreciated that any debris resulting from a procedure that is entrained by the blood flow to the patient in the direction of the filter 132 is directed into the upstream conical section 50a and retained by the polymeric membrane 40. The debris is thus not able to pass into the either the brachiocephalic artery 24, the left common carotid artery 26 and/or the left subclavian artery 28, or in to the descending aorta 18.

Figure 4 shows the filter 132 in a deployed state. The filter 132 is initially provided in an un-deployed or collapsed state whereupon it is positioned within the delivery catheter 54. The collapsed filter 132 may be covered by a sheath or sleeve that maintains the filter 132 in the collapsed state during advancement of the delivery catheter 54 through the vasculature of the patient. The sheath or sleeve may be defined by the body of the delivery catheter 54. Upon reaching the desired location within the aorta 12 of a patient, the filter 132 can be moved to the deployed state. This may be achieved by, for example, manipulating the sheath covering the filter 132 so as to uncover the filter 132 or by manipulating the filter 132 so as to extend out of the delivery catheter 54 so as to uncover the filter 132. The filter 132 may then move from the collapsed state to the deployed state by known means such as, for example, shape memory alloy portions of the filter 132.

Once the filter 132 has been deployed (i.e. positioned) into the arch of aorta 16, the TAVI delivery catheter 30 is passed through the neck portion 52 of the filter 132 prior to the TAVI procedure being conducted. After the procedure has been conducted, the TAVI catheter 30 can be removed from the patient's vasculature. The filter 132 can subsequently be moved back to its collapsed state within the delivery catheter 54, whilst retaining any debris that has been captured.

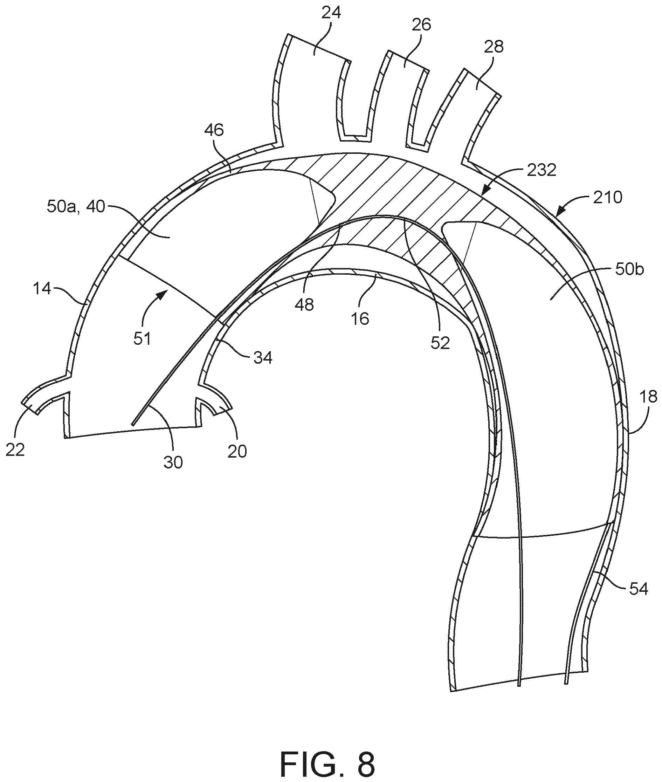

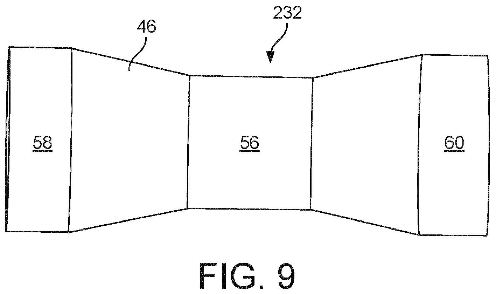

Referring now figures 8 and 9, there is shown an alternative embodiment of an embolic filter assembly generally designated 210. Features common to the filter 132 described with reference to Figures 4 and 5 are identified with like reference numerals.

The embolic filter assembly 210 includes a delivery catheter 54 and an embolic filter 232. As with the filter 132 of Figures 4 and 5, the support frame 38 defines an elongate body or tubular portion 46. The filter 232 includes an inner hourglass portion 48 which is located within the elongate body 46. The hourglass portion 48 is tubular and comprises opposing conical sections 50a, 50b which each narrow towards and are joined by a neck portion 52. The conical sections comprise an upstream conical section 50a and a downstream conical section 50b. In use, the upstream conical section 50a faces the direction of fluid flow (indicated by arrow 51) within the aorta 12. The neck portion 52 is normally closed but can open so as to allow a medical device to pass there through.

In figure 8, the delivery catheter 54 is separate from the TAVI delivery catheter 30. The TAVI delivery catheter 30 is shown passing through the neck portion 52. The neck portion 52 is configured so as to maintain contact with the TAVI delivery catheter 30 and thus not provide a fluid flow path through the filter 132 between the neck portion 52 and the TAVI delivery catheter 30.

The support frame 38 (i.e. the tubular and hourglass portions 46, 48) of the filter 232 is formed from braided nitinol mesh. The upstream conical section 50a is furthermore lined with a polymeric membrane 40. The polymeric membrane 40 may comprise a biocompatible porous fabric having a pore size of between 100 and 200 nanometers.

It will be noted that the overall length of the filter 232 is greater than that of the filter 132 described with reference to Figures 4 and 5. As can be seen from Figure 8, the filter 232, in use, extends from a position in the ascending aorta 14, through the arch of aorta 16 and into the descending aorta 18. It will further be noted that the elongate body 46 has a central section 56 that is provided between respective end sections 58, 60. The central section 56 is narrower, i.e. has a reduced outer diameter, compared to that of the respective first and second end regions 58, 60. The outer diameter of the central section 56 is less than the diameter of the aorta 12 such that, in use, the central section 56 of the outer tubular potion 46 of the filter 232 is spaced from the wall of the aorta 12 when the filter 232 is in the deployed state. The filter 232 thus contacts the aorta 12 only by the greater diameter end sections 58,60. In the embodiment shown, the end sections 58, 60 of the outer tubular potion 46 of the filter 232 contact the wall of the ascending aorta 14 and descending aorta 16 respectively. The central section 56 of the outer tubular potion 46 of the filter 232 is located in the arch of aorta 16 and is thus spaced from the junction of each of the brachiocephalic artery 24, the left common carotid artery 26 and the left subclavian artery 28 with the arch of aorta 16. This prevents the possibility of the filter 232 potentially dislodging any calcifications that might be present in this part of the aorta 12. Whilst not illustrated, it will be appreciated that the other embodiments of the embolic filter may also include such a narrower central section of the elongate body/support frame, in particular the embodiments shown in Figures 4 to 7.

As before, it will be appreciated that any debris resulting from a procedure that is entrained by the blood flow to the patient in the direction of the filter 232 is directed into the upstream conical section 50a and retained by the polymeric membrane 40. The debris 36 is thus not able to pass into the either the brachiocephalic artery 24, the left common carotid artery 26 and/or the left subclavian artery 28, or in to the descending aorta 18.

Figure 8 shows the filter 232 in a deployed state. The filter 232 is initially provided in an un-deployed or collapsed state whereupon it is positioned within the delivery catheter 54. The collapsed filter 232 may, for example, be covered by a sheath or sleeve that maintains the filter 232 in the collapsed state during advancement of the delivery catheter 54 through the vasculature of the patient. The sheath or sleeve may be defined by the body of the delivery catheter 54. Upon delivery (i.e. upon reaching the desired location within the aorta 12 of a patient), the filter 232 can be moved to the deployed state. This may be achieved by, for example, moving the sheath covering the filter 232 in a first direction so as to uncover the filter 232 or by manipulating the filter 232 so as to extend out of the delivery catheter 54 so as to uncover the filter 232. The filter 232 may then move from the collapsed state to the deployed state shape memory alloy portions of the support frame 38.

Once the filter 232 has been deployed into the arch of aorta 16, the TAVI delivery catheter 30 is passed through the neck portion 52 of the filter 232 prior to the TAVI procedure being conducted. After the procedure has been conducted, the TAVI catheter 30 can be removed from the patient's vasculature. The filter 232 can subsequently be moved back to its collapsed state within the delivery catheter 54 by movement of the sheath in a second direction opposite to the first direction, whilst retaining any debris that has been captured.

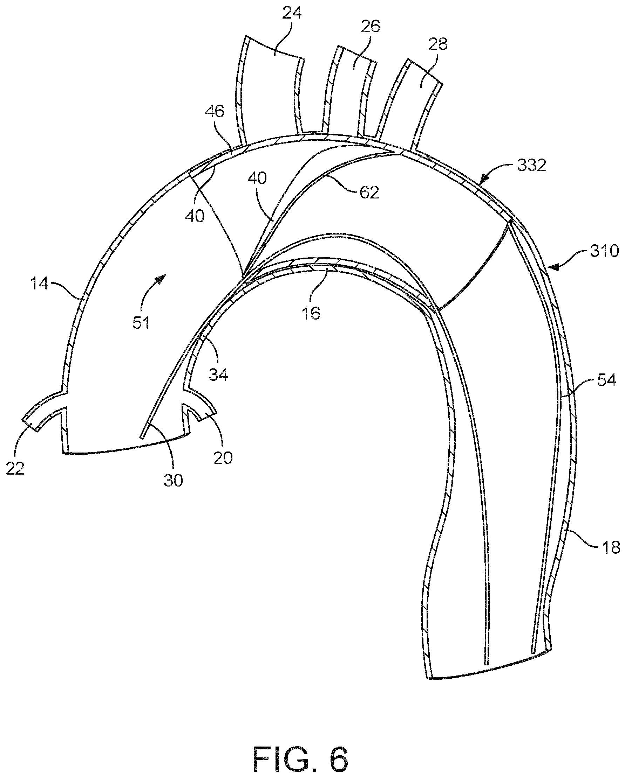

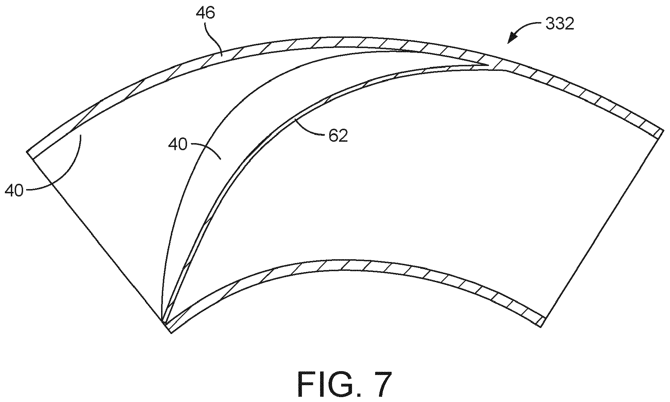

Referring now figures 6 and 7, there is shown an alternative embodiment of an embolic filter assembly generally designated 310. Features common to the filter 132 described with reference to Figures 4 and 5 are identified with like reference numerals.

The embolic filter assembly 310 includes a delivery catheter 54 and an embolic filter 132. The support frame 38 defines an elongate body 38 or an outer tubular portion 46. The filter 132 includes an inclined inner flexible leaflet 62 that extends across the interior of the body 46. The leaflet 62 is arranged to define a neck portion 52 between the leaflet 62 and the tubular portion 46. The leaflet 62 is normally closed but can open so as to allow a medical device to pass there through.

In this embodiment, the delivery catheter 54 is separate from the TAVI delivery catheter 30. The TAVI delivery catheter 30 is shown passing through the neck portion 52. The neck portion 52 is configured so as to maintain contact with the TAVI delivery catheter 30 and thus not provide a fluid flow path through the filter 132 between the neck portion 52 and the TAVI delivery catheter 30.

The leaflet 62 is arranged to define an upstream conical sections 50a that tapers towards the neck portion 52. In use, the upstream conical section 50a faces the direction of fluid flow within the aorta 12. The support frame 38 (i.e. the tubular portion 46 and flexible leaflet 62) of the filter 332 is formed from braided nitinol mesh. The flexible leaflet 62 and the part of the tubular portion 46 that is upstream of the flexible leaflet 62 (i.e. the upstream conical section 50a) is lined with a polymeric membrane 40. The polymeric membrane 40 may comprise a biocompatible porous fabric having a pore size of between 100 and 200 nanometers.

As can be readily seen in figure 6, the outer tubular portion 46 of the filter 332 is curved and is located in, and conforms to the shape of, the arch of aorta 16 in the region of the brachiocephalic artery 24, the left common carotid artery 26 and the left subclavian artery 28. More specifically, the outer tubular portion 46 extends from a location in the arch of aorta 16 that is upstream of the brachiocephalic artery 24 to a location that is downstream of the left subclavian artery 28. It will thus be appreciated that the outer tubular portion 46 overlies the junction of each of the brachiocephalic artery 24, the left common carotid artery 26 and the left subclavian artery 28 with the arch of aorta 16. It will further be appreciated that any debris resulting from a TAVI procedure that is entrained by the blood flow to the patient in the direction of the filter 132 is directed into the filter 332 and retained by the polymeric membrane 40 provided on the flexible leaflet 62 and the part of the tubular portion 46 that is upstream of the flexible leaflet 62. The debris is thus not able to pass into the either the brachiocephalic artery 24, the left common carotid artery 26 and/or the left subclavian artery 28, or in to the descending aorta 18.

Figure 6 shows the filter 332 in a deployed state. The filter 332 is initially provided in an un-deployed or collapsed state whereupon it is positioned within the delivery catheter 54. The collapsed filter 332 may, for example, be covered by a sheath or sleeve that maintains the filter 332 in the collapsed state during advancement of the delivery catheter 54 through the vasculature of the patient. The sheath or sleeve may be defined by the body of the delivery catheter 54. Upon reaching the desired location within the aorta 12 of a patient, the filter 332 can be moved to the deployed state. This may be achieved by, for example, moving the sheath covering the filter 332 in a first direction, i.e. a retraction direction, so as to uncover the filter 332 or by manipulating the filter 332 so as to extend out of the delivery catheter 54 so as to uncover the filter 332. The filter 332 may then move from the collapsed state to the deployed state by shape memory alloy portions of the support frame 38.

Once the filter 332 has been deployed (i.e. positioned) into the arch of aorta 16, the TAVI delivery catheter 30 is passed through the neck portion 52 of the filter 332 prior to the TAVI procedure being conducted. After the procedure has been conducted, the TAVI catheter 30 can be removed from the patient's vasculature. The filter 332 can subsequently be moved back to its collapsed state within the delivery catheter 54 by relative movement between the filter 332 and the sheaf in a second direction, opposite to the first direction, whilst retaining any debris that has been captured.

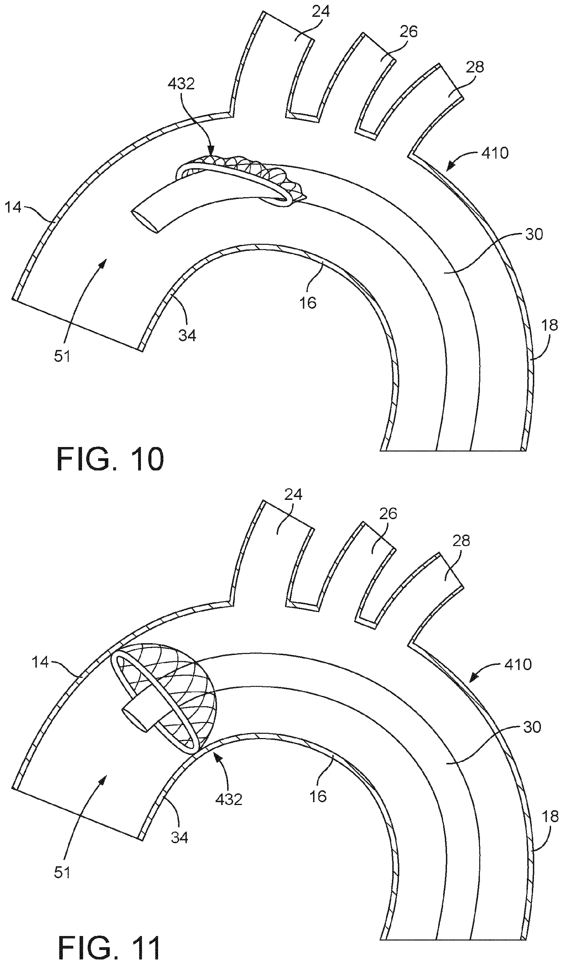

Referring now to figures 10, 11 and 12 there is shown an alternative embodiment of an embolic filter assembly generally designated 410. The embolic filter assembly 410 is a combined delivery catheter and embolic filter located within the aorta 12 of a patient. The features of the aorta include the ascending aorta 14, the arch of aorta 16, the descending aorta 18, the brachiocephalic artery 24, the left common carotid artery 26 and the left subclavian artery 28.

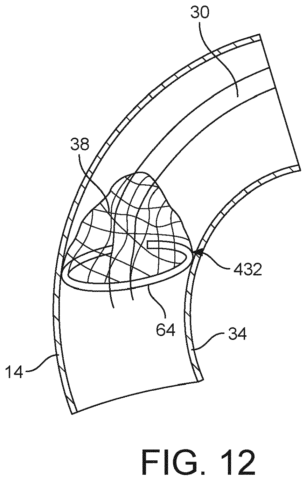

The embolic filter assembly includes a TAVI delivery catheter 30 and an embolic filter generally designated 432. Figure 10 shows the filter 432 in its collapsed state (with the sheath not shown for clarity), whereas Figures 11 and 12 show the filter 432 in its deployed state.

The filter 432 is provided circumferentially around an outer surface of the TAVI delivery catheter 30 and extends across the annulus defined the between the TAVI delivery catheter 30 and the wall 34 of the ascending aorta 14. The filter 432 is configured so as to be able to conform to the wall 34 of the ascending aorta 14 and thus provide required sealing characteristics against the wall of ascending aorta 14. The support frame 38 comprises a nitinol spiral ring, coil, or arm 64. The frame 38 may include a braided nitinol mesh that is connected between the spiral ring 64 and the TAVI delivery catheter 30, and (not shown) a polymeric membrane. The polymeric membrane may comprise a biocompatible porous fabric having a pore size of between 100 and 200 nanometers. The polymeric membrane is fixed to the frame 38 by, for example, adhesive.

In the embodiment shown, the filter 432 is positioned in the ascending aorta 14 upstream of the arch of aorta 16. Any debris that may be dislodged by the TAVI procedure is thus captured by the filter 432 and prevented from reaching and entering the brachiocephalic artery 24, the left common carotid artery 26 and/or the left subclavian artery 28.

The filter 432 is initially provided in an un-deployed or collapsed state whereupon it conforms closely to the outer surface of the TAVI delivery catheter 30 as shown in figure 10. The collapsed filter 432 may, for example, be covered by a sheath that maintains the filter 32 in the collapsed state during advancement of the TAVI delivery catheter 30 through the vasculature of the patient. Upon delivery (i.e. upon reaching the desired location within the aorta 12 of a patient), the filter 432 can be moved to the deployed state. This may be achieved by, for example, moving the sheath covering the filter 432 in a first, e.g. retraction, direction so as to uncover the filter 432. The filter 432 may then move from the collapsed state to the deployed state by expansion of the nitinol spiral ring 64. As can be seen in figures 11 and 12, the spiral ring or coil 64 expands so as to contact the wall 34 of the ascending aorta 14.

With the filter 432 deployed and extending across the annulus defined between the TAVI delivery catheter 30 and the wall 34 of the ascending aorta 14, the TAVI procedure can be conducted. As noted above, any debris that is be dislodged by the TAVI procedure is thus captured by the filter 432 and prevented from reaching and entering the brachiocephalic artery 24, the left common carotid artery 26 and/or the left subclavian artery 28. Once the TAVI procedure has been completed, and prior to the withdrawal of the TAVI delivery catheter 30 from the vasculature of the patient, the filter 432 is moved back to its un-deployed or collapsed state from its deployed state while still retaining any debris that may have been collected.

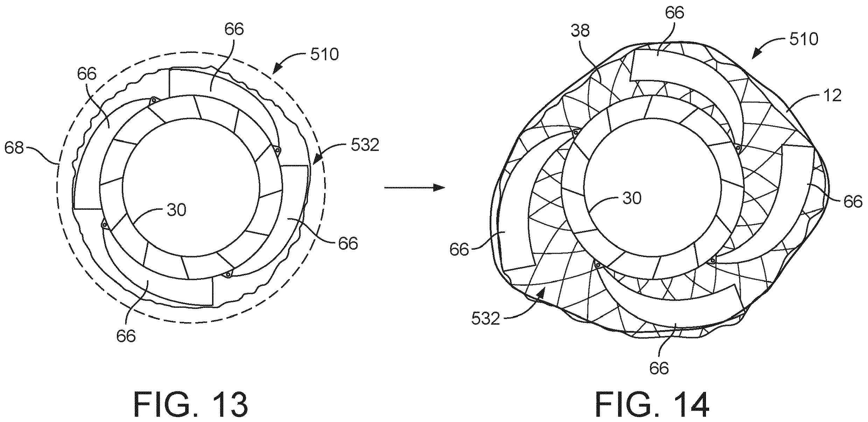

Referring now to Figures 13 and 14 there is shown an alternative embodiment of an embolic filter assembly generally designated 510. The embolic filter assembly 510 is a combined delivery catheter and embolic filter located within the aorta 12 of a patient.

The embolic filter assembly 510 includes a TAVI delivery catheter 30 and an embolic filter generally designated 532. Figure 13 shows the filter 532 in its collapsed state, whereas Figure 14 shows the filter 532 in its deployed state.

The filter 532 is provided circumferentially around an outer surface of the TAVI delivery catheter 30 and extends across the annulus defined the between the TAVI delivery catheter 30 and the wall of the aorta 12. The filter 532 is configured so as to be able to conform to the wall of the aorta 12 and thus provide required sealing characteristics against the wall of the aorta 12. The support frame 38 comprises a plurality nitinol arms 66. The support frame 38 may include a braided nitinol mesh that is connected between the nitinol arms 66 and the TAVI delivery catheter 30. A polymeric membrane (not shown) covers the support frame 38. The polymeric membrane may comprise a biocompatible porous fabric having a pore size of between 100 and 200 nanometers. The polymeric membrane is fixed to the frame 38 by, for example, adhesive.

The filter 532 is initially provided in an un-deployed or collapsed state whereupon it conforms closely to the outer surface of the TAVI delivery catheter 30 as shown in figure 13. The collapsed filter 532 is covered by a sheath 68 that maintains the filter 532 in the collapsed state during advancement of the TAVI delivery catheter 30 through the vasculature of the patient. As can be seen from Figure 13, the nitinol arms 66 are maintained in position whereupon they rest against the TAVI delivery catheter 30.

Upon delivery (i.e. upon reaching the desired location within the aorta 12 of a patient), the filter 532 can be moved to the deployed state. This may be achieved by moving the sheath 68 covering filter 532 in a first, i.e. retraction, direction so as to uncover the filter 532. The filter 532 may then move from the collapsed state to the deployed state by expansion of the support frame 38. In particular, the filter 532 may then move from the collapsed state to the deployed state by expansion of the nitinol spiral ring 64. As can be seen in figure 14, the nitinol arms 66 are able move outwardly from the TAVI delivery catheter so as to contact the wall of the aorta 12.

With the filter 532 deployed and extending across the annulus defined between the TAVI delivery catheter 30 and the wall of the aorta 12, the TAVI procedure can be conducted. As noted above, any debris that is be dislodged by the TAVI procedure is thus captured by the filter 532 and prevented from reaching and entering the brachiocephalic artery 24, the left common carotid artery 26 and/or the left subclavian artery 28. Once the TAVI procedure has been completed, and prior to the withdrawal of the TAVI delivery catheter 30 from the vasculature of the patient, the filter 532 is retrieved or moved back to its un-deployed or collapsed state from its deployed state while still retaining any debris that may have been collected.

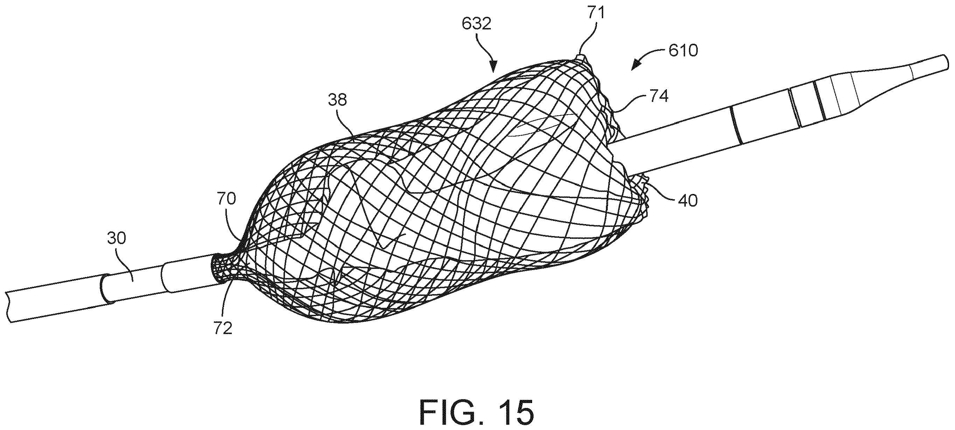

Referring now to Figure 15 there is shown an alternative embodiment of an embolic filter assembly generally designated 610. The embolic filter assembly 610 is a combined delivery catheter and embolic filter located within the aorta 12 of a patient.

The embolic filter assembly 610 includes a TAVI delivery catheter 30 and an embolic filter generally designated 632. Figure 15 shows the filter 632 in its deployed state. The filter 632 is provided circumferentially around an outer surface of the TAVI delivery catheter 30 and, in use, extends across the annulus defined the between the TAVI delivery catheter 30 and the wall of the aorta. The filter 632 is configured so as to be able to conform to the wall of the aorta and thus provide required sealing characteristics against the wall of the aorta.

The support frame 38 include an elongate, e.g. tubular, body. The body 38 has a proximal end 70 and a distal end 71 that is formed from a braided nitinol mesh, and a polymeric membrane 40 covering the support frame 38. The tubular body 38 defines an internal volume, and the polymeric membrane 40 is arranged in the internal volume of the elongate braised nitinol mesh 38. The tubular frame 38 is connected at its proximal end 70 to the TAVI delivery catheter 30. The polymeric membrane comprises a biocompatible porous fabric having a pore size of between 100 and 200 nanometers.

The polymeric membrane 40 has a proximal, or downstream, end 72 and a distal, or upstream, end 74. The polymeric membrane 40 is arranged to cover the braised nitinol mesh 38 at the upstream end region 74 of the braided nitinol mesh 38, and is connected to said braided nitinol mesh at the downstream end region 72 of the braided nitinol mesh 38. The proximal end 72 of the polymeric membrane 40 is attached to the TAVI delivery catheter 30 at the same location as the proximal end 70 of the tubular frame 38. The attachment of the proximal end 72 of the polymeric membrane 40 to the TAVI delivery catheter 30 may achieved by adhesive, sewing, crimping or thermobonding. The distal end 74 of the polymeric membrane 40 is connected to the distal end 71 of the tubular frame 38. The attachment of the polymeric membrane 40 to the tubular frame 38 may be achieved by sewing the membrane 40 to the mesh structure. Alternatively, the membrane 40 may be wrapped over the tubular frame 38 and thereafter glued or thermobonded to the frame 38.

Attachment of the polymeric membrane 40 to the frame 38 and TAVI delivery catheter 30 at opposing ends enables the membrane 40 to be readily moved between the deployed and collapsed states of the filter 632. The polymeric membrane 40 is provided with an elongate length that is greater than the elongate length of the braised nitinol mesh 38. In this way, the polymer membrane 40 provides loose material along its length (i.e. between the upstream 74 and downstream 72 end regions of the mesh 38), which facilitates crimping of the nitinol mesh 38 to a delivery catheter 30.

The filter 632 is initially provided in an un-deployed or collapsed state whereupon it conforms closely to the outer surface of the TAVI delivery catheter 30. The collapsed filter 632 may covered by a sheath that maintains the filter 632 in the collapsed state during advancement of the TAVI delivery catheter 30 through the vasculature of the patient.

Upon reaching the desired location within the aorta of a patient, the filter 632 can be moved to the deployed state. This may be achieved by, for example, moving the sheath covering filter 632 in a first, e.g. retraction, direction so as to uncover the filter 632. The filter 632 may then move from the collapsed state to the deployed state by expansion of the support frame 38.

With the filter 632 deployed and extending across the annulus defined between the TAVI delivery catheter 30 and the wall of the aorta, the TAVI procedure can be conducted. As noted above, any debris that is be dislodged by the TAVI procedure is thus captured by the filter 632 and prevented from reaching and entering the brachiocephalic artery 24, the left common carotid artery 26 and/or the left subclavian artery 28. Once the TAVI procedure has been completed, and prior to the withdrawal of the TAVI delivery catheter 30 from the vasculature of the patient, the filter 632 is moved back to its un-deployed or collapsed state from its deployed state by relative movement between the filter 632 and the sheath in a second direction, opposite to the first direction, while still retaining any debris that may have been collected.

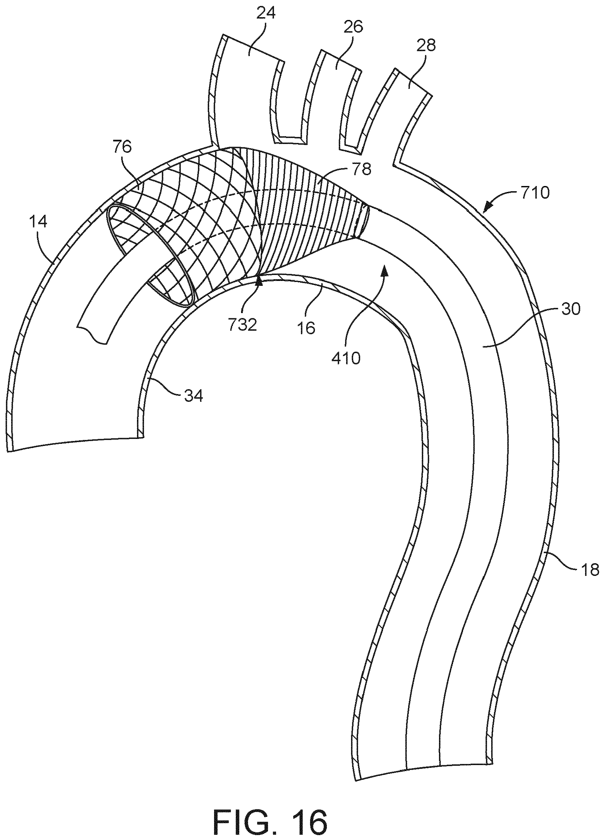

Referring now to Figure 16 there is shown an alternative embodiment of an embolic filter assembly generally designated 710. The embolic filter assembly 710 is a combined delivery catheter and embolic filter located within the aorta 12 of a patient. The features of the aorta include the ascending aorta 14, the arch of aorta 16, the descending aorta 18, the brachiocephalic artery 24, the left common carotid artery 26 and the left subclavian artery 28.

The embolic filter assembly 610 includes a TAVI delivery catheter 30 and an embolic filter generally designated 732. Figure 16 shows the filter 732 in its deployed state.

The filter 732 is provided circumferentially around an outer surface of the TAVI delivery catheter 30 and extends across the annulus defined the between the TAVI delivery catheter 30 and the wall 34 of the ascending aorta 14. The filter 732 is configured so as to be able to conform to the wall 34 of the ascending aorta 14 and thus provide required sealing characteristics against the wall of ascending aorta 14. The filter 432 comprise a tubular frame 76 formed from a braided nitinol mesh and a frusto-conical polymeric membrane 78. The polymeric membrane 78 is connected at one end to the tubular frame 76 and at the opposing end to the TAVI delivery catheter 30.

The polymeric membrane 78 may comprise a biocompatible porous fabric having a pore size of between 100 and 200 nanometers. The polymeric membrane 78 is fixed to the tubular frame 76 by, for example, sewing, adhesive or themobonding, and to the TAVI delivery catheter 30 by adhesive, sewing, crimping or thermobonding.

In the embodiment shown, the filter 732 is positioned in the ascending aorta 14 upstream of the arch of aorta 16. Any debris that may be dislodged by the TAVI procedure is thus captured by the filter 732 and prevented from reaching and entering the brachiocephalic artery 24, the left common carotid artery 26 and/or the left subclavian artery 28.

The filter 732 is initially provided in an un-deployed or collapsed state whereupon it conforms closely to the outer surface of the TAVI delivery catheter 30. The collapsed filter 732 may, for example, be covered by a sheath that maintains the filter 732 in the collapsed state during advancement of the TAVI delivery catheter 30 through the vasculature of the patient. Upon reaching the desired location within the aorta 12 of a patient, the filter 732 can be moved to the deployed state. This may be achieved by, for example, manipulating the sheath covering filter 732 so as to uncover the filter 732. The filter 732 may then move from the collapsed state to the deployed state by expansion of the tubular frame 76.

With the filter 732 deployed and extending across the annulus defined between the TAVI delivery catheter 30 and the wall 34 of the ascending aorta 14, the TAVI procedure can be conducted. As noted above, any debris that is be dislodged by the TAVI procedure is thus captured by the filter 732 and prevented from reaching and entering the brachiocephalic artery 24, the left common carotid artery 26 and/or the left subclavian artery 28. Once the TAVI procedure has been completed, and prior to the withdrawal of the TAVI delivery catheter 30 from the vasculature of the patient, the filter 732 is moved back to its un-deployed or collapsed state from its deployed state while still retaining any debris that may have been collected.

## Claims

1. An embolic filter (32,132,232,332,432,532,632,732) comprising:
a support frame (38,76); and
a porous polymeric membrane connected to the support frame,
wherein the embolic filter is configured to be movable between a collapsed state and a deployed state where the filter (32,432,532,632,732) extends, in use, into contact with an inner wall of a patient's vasculature,
wherein the support frame (38,76) comprises a shape memory material such that the support frame acts as a deployment arrangement to move the embolic filter from the collapsed state into the deployed state.

2. An embolic filter (32,132,232,332,432,532,632,732) according to claim 1, comprising a nitinol support frame (38,76).

3. An embolic filter (32,132,232,332,432,532,632,732) according to claim 1 or claim 2, wherein the embolic filter comprises a neck portion configured to conform to an external surface of a catheter body (31), in use.

4. An embolic filter (32,132,232,332,432,532,632,732) according to claim 3, wherein the support frame (38,76) comprises the neck portion and a plurality of flexible petals extend from said neck portion, and wherein a distal end of each of the flexible petals are configured, in use, to contact with an inner wall of a patient's vasculature when in the deployed state.

5. An embolic filter (32,132,232,332,432,532,632,732) according to claim 3, comprising an internal leaflet extending across an interior of the support frame (38,76) and configured and arranged to define the neck portion.

6. An embolic filter (32,132,232,332,432,532,632,732) according to any of claims 3 to 5, wherein the embolic filter defines an inlet region at an upstream end of the embolic filter that is covered with the porous polymeric membrane, and wherein said inlet region tapers towards the neck portion, e.g. so as to define a substantially conical inlet region.

7. An embolic filter (32,132,232,332,432,532,632,732) according to any one of claims 3 to 6, wherein the support frame (38,76) comprises a nitinol tube or ring, e.g. a braided nitinol mesh, configured, in use, to contact with an inner wall of a patient's vasculature when in the deployed state, wherein a first end or region of the porous polymeric membrane is attachable to a body of a delivery catheter, in use, and a second end or region of the porous polymeric membrane is attached to the support frame such that the porous polymeric membrane forming an inlet region which tapers towards the first end or region of the porous polymeric membrane to define an inlet region which tapers towards the neck portion.

8. An embolic filter (32,132,232,332,432,532,632,732) according to any preceding claim, wherein the support frame (38,76) defines an elongate body comprising first and second end regions thereof each region configured, in use, to contact with an inner wall of a patient's vasculature when in the deployed state.

9. An embolic filter (32,132,232,332,432,532,632,732) according to claim 8, wherein the elongate body comprises an external surface, said external surface comprising a narrower region between the first and second end regions.

10. An embolic filter (32,132,232,332,432,532,632,732) according to any preceding claim, wherein a first end or region of the porous polymeric membrane is attachable to a body of a delivery catheter, in use, and wherein a second end or region of the membrane is attached to the support frame (38,76), wherein the support frame (38,76) comprises a ring, a coil or an arm configured to extend at least partially around a body of a delivery catheter, in use, over which the porous polymeric membrane extends.

11. An embolic filter (32,132,232,332,432,532,632,732) according to any preceding claim, wherein the support frame (38,76) is formed from an elongate tubular braided nitinol mesh connectable to a body of a delivery catheter, in use, and defining an internal volume.

12. An embolic filter (32,132,232,332,432,532,632,732) according to claim 11, wherein the porous polymeric membrane is positioned in the internal volume of the elongate braised nitinol mesh, and wherein the porous polymeric membrane is arranged to cover the braised nitinol mesh at a first, upstream, end region thereof and is connected to said braided nitinol mesh at a second, downstream, end region thereof.

13. An embolic filter (32,132,232,332,432,532,632,732) according to claim 12, wherein the porous polymeric membrane comprises an elongate length that is greater than the elongate length of the braised nitinol mesh.

14. An embolic filter (32,132,232,332,432,532,632,732) according to any preceding claim, wherein the porous polymeric membrane (40,78) is a biocompatible fabric comprising a pore size of between 100 and 200 nanometers.

15. An embolic filter assembly comprising:
a delivery catheter; and
an embolic filter (32,132,232,332,432,532,632,732) according to any preceding claim integrated with the delivery catheter,
wherein the embolic filter is deployable from the delivery catheter prior to the delivery of the replacement valve and retrievable to the delivery catheter after delivery of the replacement valve,
wherein the filter (32,132,232,332,432,532,632,732) is retained in the collapsed state by an overlying sleeve (68).
